(19) Europäisches Patentamt

European Patent Office

Office européen des brevets

(11) **EP 0 576 879 B1**

(12) **EUROPÄISCHE PATENTSCHRIFT**

(45) Veröffentlichungstag und Bekanntmachung des Hinweises auf die Patenterteilung:
**21.10.1998 Patentblatt 1998/43**

(51) Int Cl.$^6$: **G01N 33/53**, G01N 21/47, G01N 21/27

(21) Anmeldenummer: **93109189.6**

(22) Anmeldetag: **08.06.1993**

(54) **Verfahren zur analytischen Bestimmung der Konzentration eines Bestandteils einer medizinischen Probe**

Process for the analytical determination of the concentration of a part of a medical specimen

Procédé pour le détermination analytique de la concentration d'un composant d'un échantillon médicinal

(84) Benannte Vertragsstaaten:
**AT BE CH DE DK ES FR GB GR IE IT LI LU NL PT SE**

(30) Priorität: **03.07.1992 DE 4221807**

(43) Veröffentlichungstag der Anmeldung:
**05.01.1994 Patentblatt 1994/01**

(73) Patentinhaber: **BOEHRINGER MANNHEIM GMBH 68298 Mannheim (DE)**

(72) Erfinder:
- **Schäfer, Rainer, Dr.
  D-8000 München 60 (DE)**
- **Berding, Christoph, Dr.
  D-8000 München 80 (DE)**
- **Lang, Fridl, Dr.
  D-8132 Tutzing (DE)**
- **Kleider, Wilhelm
  D-8110 Murnau (DE)**
- **Wolf, Peter, Dr.
  D-8121 Habach (DE)**

(74) Vertreter: **Pfeifer, Hans-Peter, Dr.,
Dr. H.-P. Pfeifer Dr. P. Jany, Patentanwälte
Beiertheimer Allee 19
76137 Karlsruhe (DE)**

(56) Entgegenhaltungen:
**EP-A- 0 148 463          FR-A- 2 353 860**

- **L.FAHRMEIR UND ALFRED HAMERLE
  'Multivariate statistische Verfahren' 1984 ,
  WALTER DE GRUYTER , BERLIN,NEW YORK *
  Seite 301 - Seite 302 * * Seite 310 - Seite 316 ***

**Beschreibung**

Die Erfindung betrifft ein Verfahren zur analytischen Bestimmung der Konzentration C eines Bestandteiles einer medizinischen Probe, bei dem eine Reaktion der Probe mit Reagenzien zu einer zeitabhängigen Änderung S(t) ("Kinetik") einer Meßgröße S führt und die Konzentration C gemäß einer Auswertekurve C=f(X) mit einer aus S(t) abgeleiteten Eingangsvariablen X korreliert, wobei die zu der Auswertekurve C=f(X) inverse Kalibrationskurve $X=f^{-1}(C)$ nicht monoton ist, so daß der gleiche Wert der Eingangsvariablen X auf mindestens zwei Teilbereichen der Kalibrationskurve unterschiedlichen Werten der Konzentration C entspricht, und die Auswertekurve mindestens für einen Teil der möglichen X-Werte mehrdeutig ist.

In der Analysetechnik, insbesondere bei der Analyse von Körperflüssigkeiten wie Blut und Urin sind Verfahren weit verbreitet, die auf einer spezifischen Bindungsreaktion zweier bioaffiner Bindungspartner basieren. Spezifische Bindungsreaktionen in diesem Sinne sind insbesondere immunologische Interaktionen, also Wechselwirkungen zwischen Antigenen bzw. Haptenen einerseits und Antikörpern andererseits. Andere Beispiele sind die Bindungsreaktionen Lektin-Zucker, Biotin-Streptavidin oder Wirkstoff-Rezeptor-Wechselwirkungen. Im folgenden wird ohne Beschränkung der Allgemeinheit beispielhaft auf immunologische Wechselwirkungen Bezug genommen.

Solche Analyseverfahren zeichnen sich durch hohe Spezivität und Nachweisempfindlichkeit aus. Ein seit langem bekanntes Problem immunologischer Analyseverfahren besteht jedoch darin, daß die Auswertekurve C=f(X) in vielen Fällen nicht eindeutig ist. Dieses Phänomen wird auch als "(high dose) Hook-Effekt" bezeichnet. Es wird sowohl bei heterogenen als auch bei homogenen immunologischen Nachweisverfahren beobachtet.

Bei heterogenen Ein-Schritt-Sandwich-Testen kommt es beispielsweise bei einem hohen Überschuß des Probenantigens zu einer Absättigung sowohl des festphasengebundenen Antikörpers als auch des markierten Antikörpers. Hieraus resultiert eine Verminderung der Kopplung des markierten Antikörpers mit dem festphasengebundenen Antikörper und infolgedessen eine Reduzierung des Meßsignals mit steigender Analytkonzentration.

Ein wichtiges Beispiel homogener auf spezifischen Bindungsreaktionen basierender Analyseverfahren sind Reaktionen, die zu Molekülaggregaten, Makromolekülen oder Vernetzungen mit kleinen Partikeln, wie beispielsweise Latexpartikeln, Dextranen, Liposomen oder Metallsuspensionen führen. Diese Aggregatbildung führt zu einer Änderung des Streulichtverhaltens, das sich mit einem geeigneten physikalischen Meßverfahren nachweisen läßt. Gebräuchlich ist die Bestimmung der durch die Trübung verursachten Extinktion (Turbidimetrie) sowie die Messung der Lichtstreuung (Nephelometrie).

Bei solchen Tests läßt sich der nichtmonotone Verlauf der Kalibrationskurve $X=f^{-1}(C)$, der zu der beschriebenen Doppeldeutigkeit der Eingangsvariablen X führt, dadurch erklären, daß bei hohen Konzentrationen des Probenantigens die Bindungsstellen der an der Reaktion beteiligten Antikörper zunehmend von Antigen abgesättigt werden. Infolgedessen nimmt bei hohem Antigen-Überschuß die Vernetzung der Primärteilchen nicht mehr zu, sondern sie nimmt ab und die Trübung vermindert sich.

Dieser auch als "Antigenüberschuß-Phänomen" bezeichnete Effekt wurde bereits 1935 von Heidelberger und Kendall erkannt. Man bezeichnet die nicht monotone Kalibrationskurve daher häufig als "Heidelberger-Kurve". Eine eingehendere Darstellung der Problematik ist beispielsweise der europäischen Patentschrift 0 148 463 zu entnehmen, worauf hier Bezug genommen wird.

In dieser Druckschrift findet sich auch eine ausführliche Diskussion vorbekannter Verfahren, um dem Problem der Zweideutigkeit der Heidelberger-Kurven zu begegnen. Sie seien an dieser Stelle nur kurz zusammengefaßt.

Weit verbreitet ist die Doppelbestimmung mit zwei verschiedenen Probenverdünnungen. Wenn der Test für die verdünnte Probe eine scheinbar höhere Konzentration anzeigt, liegt die gemessene Probenkonzentration im fallenden Teil der Auswertekurve. Es wird dann üblicherweise so lange weiterverdünnt, bis für zwei aufeinanderfolgende Verdünnungen jeweils abnehmende Konzentrationen erhalten werden.

Statt der Untersuchung verschieden stark verdünnter Proben kann die Konzentration der Probe auch durch nachträgliche Zugabe von Probenflüssigkeit variiert werden. Eine weitere Alternative besteht darin, nach Ablauf einer ersten Reaktion zusätzlichen Antikörper zuzugeben, um aus der dann erhaltenen Eingangsvariablen darauf zu schließen, ob die zu bestimmende Konzentration im ansteigenden oder im abfallenden Teil der Heidelberger-Kurve liegt. Nachteilig ist bei jedem dieser Verfahren, daß aufwendige zusätzliche Handhabungsschritte erforderlich sind. Sie lassen sich deswegen nicht ohne weiteres auf üblichen Analyseautomaten realisieren. Außerdem erhöht die zusätzliche Messung die Meßzeit und reduziert damit den Probendurchsatz des Analyseautomaten.

Vielfach wird deshalb versucht, durch Einsatz einer großen Antikörpermenge sicherzustellen, daß sämtliche praktisch vorkommenden Konzentrationen des Analyten im "gültigen Meßbereich", d.h. im steigenden Teil der Heidelberger-Kurve liegen. Die hohe Konzentration des Antikörpers führt jedoch im Bereich relativ geringer Analytkonzentrationen zu einem sehr flachen Verlauf der Kalibrationskurve $X=f^{-1}(C)$ und infolgedessen zu einer reduzierten Analysepräzision. Außerdem werden die Kosten des Tests durch die große Antikörpermenge wesentlich erhöht. Bei einigen Analyten, die in sehr hohen Konzentrationen oder in einem sehr großen Konzentrationsbereich analysiert werden müssen, ist dieses Verfahren praktisch nicht realisierbar.

In der deutschen Patentschrift 27 24 722 ist ein immunonephelometrisches Verfahren beschrieben, welches auf der Bestimmung der zeitlichen Änderung (Kinetik) der Trübung basiert und ohne zusätzlichen zeitlichen Aufwand eine eindeutige Zuordnung des Meßwertes zum ansteigenden bzw. abfallenden Teil der Kalibrationskurve erlauben soll. In der Anfangszeit der Trübungsmessungen hatte man Endpunktbestimmungen durchgeführt, d.h. als Eingangsvariable X wurde der konstante Wert der Meßgröße S verwendet, der sich am Ende der zu der Trübung führenden Aggregationsreaktion asymptotisch einstellt. Einige Jahre vor dem Anmeldetag der deutschen Patentschrift 27 24 722 war vorgeschlagen worden, die bei der Endpunktbestimmung erforderliche lange Meßzeit durch eine kinetische Messung zu verkürzen. Dieser Vorschlag basiert auf der Erkenntnis, daß die maximale Änderungsgeschwindigkeit der Meßgröße S ($dS/dt_{max}$), welche auch als $V_{max}$ bezeichnet wird, als Eingangsvariable X verwendet werden kann, welche in einer für die quantitative Analyse ausreichenden Genauigkeit mit der Konzentration C des Analyt-Antigens korreliert.

Auch dieser Zusammenhang C=f(X) ist jedoch zweideutig. In der deutschen Patentschrift 27 24 722 wird vorgeschlagen, die Doppeldeutigkeit dieser Auswertekurve mit Hilfe einer Funktion der Meßgröße $V_{max}$ zu beseitigen, welche je nach dem jeweiligen Überschußzustand charakteristisch unterschiedliche Werte besitzen soll. Insbesondere wird die Zeit bis zum Auftreten der maximalen Änderungsgeschwindigkeit $V_{max}$ des Trübungssignals oder die zeitliche Ableitung von $V_{max}$ $(dV/dt)_{max} = (d^2S/dt^2)_{max}$ als geeignet für die Diskriminierung der Teilbereiche der Auswertekurve angesehen. In dieser Literaturstelle wird jedoch festgestellt, daß die an sich gewünschte eindeutige Bestimmung eines einzelnen Konzentrationswertes in der Praxis auf dieser Basis nicht möglich ist, weil kein einzelner Zeitwert existiert, bei dem der Antikörperüberschußkurventeil auf der einen Seite dieses Wertes und der Antigenüberschuß-Kurventeil auf der anderen Seite liegt (Spalte 21, Zeile 61 bis 68). Dieses Problem soll durch eine Koordinatentransformation und die Einführung neuer Variablen gelöst werden. Falls auf dieser Basis ein Antigenüberschuß-Zustand festgestellt wird, wird die Probe verdünnt und die Messung wiederholt.

Um diese Nachteile zu vermeiden wird in der europäischen Patentschrift 0 148 463 vorgeschlagen, den funktionalen Zusammenhang zwischen der Konzentration C, der maximalen Änderungsgeschwindigkeit des Meßsignals $V_{max}$ und der Zeit vom Reaktionsbeginn bis zum Auftreten der maximalen Reaktionsgeschwindigkeit $t_{max}$ mit einem Standardpräparat empirisch zu ermitteln und sowohl $V_{max}$ als auch $t_{max}$ an der Probe zu messen, wobei von den zwei aus einer dieser Eingangsvariablen ermittelbaren Konzentrationen mit Hilfe der zweiten Eingangsvariablen das richtige Ergebnis ermittelt wird.

Diese bekannten Verfahren gehen davon aus, daß die Zuordnung zu den Teilbereichen der doppeldeutigen Auswertekurve C=f(X) zuverlässig mit Hilfe einer bestimmten aus S(t) abgeleiteten zusätzlichen Eingangsvariablen möglich ist. Sie sind demzufolge nur in solchen Fällen anwendbar, in denen eine derartige Größe mit ausreichendem diskriminierendem Potential gefunden werden kann. Außerdem sind diese vorbekannten Verfahren davon abhängig, daß der Verlauf von S(t) kontinuierlich oder zumindest in sehr engen zeitlichen Abständen (quasikontinuierlich) bestimmt wird. Dies ist in der Praxis mit vielen Analysegeräten nicht möglich, weil diese aufgrund ihres Konstruktionsprinzips die erforderliche turbidimetrische oder nephelometrische Messung nur zu diskreten Meßzeitpunkten mit relativ großen zeitlichen Abständen erlauben. Dies gilt insbesondere für die weit verbreiteten Analysegeräte, die mit einem sich schrittweise drehenden Reaktionsrotor ausgestattet sind, wobei eine Messung des Inhaltes eines der am Rand des Rotors aufgereihten Reaktionsgefäße jeweils nur dann möglich ist, wenn sich dieses gegenüber einer Meßstation befindet.

Auf dieser Basis liegt der Erfindung die Aufgabe zugrunde, ein Verfahren zu schaffen, mit dem die Analyse im Falle einer mehrdeutigen Auswertekurve C=f(X) mit hoher Zuverlässigkeit und bei einem breiten Spektrum unterschiedlicher Tests ohne zusätzliche Handhabungsschritte bei der Probenanalyse möglich ist.

Die Aufgabe wird bei einem Verfahren der eingangs bezeichneten Art dadurch gelöst, daß folgende Schritte durchgeführt werden, um eine Eingangsvariable X einem der Teilbereiche zuzuordnen und dadurch eine eindeutige Korrelation zu einer bestimmten Konzentration C zu erreichen:

a. In einem Trainingslauf wird mindestens einmal ein Diskriminationsalgorithmus mit nachfolgenden Schritten durchgeführt

a.a S(t) wird an mehreren Kalibratoren bekannter Konzentrationen $C_j=C_1...C_m$ bestimmt, wobei die Konzentrationen $C_j=C_1...C_m$ in zwei Teilbereichen der Kalibrationskurve $X=f^{-1}(C)$ liegen;

a.b aus den Messungen von S(t) wird nach vorbestimmten Diskriminator-Generierungsverfahren ein Diskriminatorensatz generiert;

a.c aus dem Diskriminatorensatz wird mit einem Verfahren der multivariaten Statistik je ein Score für jede der Konzentrationen $C_j=C_1...C_m$ der Kalibratoren berechnet;

a.d es wird geprüft, ob sich die Scores für die Konzentrationen $C_j=C_1...c_m$ der Kalibratoren

mittels eines Grenzscores in getrennte Teilmengen teilen lassen, bei denen die Konzentrationen den Teilbereichen der Kalibrationskurve richtig zugeordnet sind;

b. In dem Trainingslauf wird der die Schritte a.a bis a.d umfassende Diskriminationsalgorithmus mit verschiedenen, nach unterschiedlichen Generierungsverfahren aus den Messungen von S(t) generierten Diskriminatorensätzen und/oder unterschiedlichen Verfahren der multivariaten Statistik wiederholt, um einen operativen Diskriminationsalgorithmus festzulegen, der-in dem Schritt a.d eine für praktische Zwecke ausreichend vollständige Trennung der Teilbereiche ermöglicht;

c. in einem Analyselauf wird:

c.a S(t) an einer zu analysierenden Probe bestimmt;
c.b daraus nach dem operativen Diskriminationsalgorithmus ein Analysescore berechnet;
c.c der Analysescore mit dem Grenzscore verglichen und
c.d die Eingangsvariable X und daraus die Konzentration C bestimmt, wobei der Wert X einer der Teilkurven der Kalibrationskurve $X=f^{-1}(C)$ durch Vergleich des Analysescore mit dem Grenzscore zugeordnet wird.

Als Diskriminator-Generierungsverfahren werden Verfahren bezeichnet, durch die in einer Folge von einem oder mehreren Schritten in genau definierter Weise durch Selektion und in den meisten Fällen durch zusätzliche Kombination von zu bestimmten Meßzeitpunkten $t_i$ gemessenen Meßwerten $S(t_i)$ Zahlenwerte abgeleitet werden, die sich als Diskriminatoren eignen. Im einfachsten Fall besteht also ein Diskriminator-Generierungsverfahren nur aus einem Selektionsschritt, nämlich der Auswahl eines bestimmten Meßwertes $S(t_i)$. Meist werden jedoch aus mehreren $S(t_i)$-Werten nach definierten mathematischen Verfahren, die nachfolgend auch als Ableitverfahren bezeichnet werden, Diskriminatoren abgeleitet.

Der Diskriminationsalgorithmus der Schritte a.a bis a.d wird so lange wiederholt, bis ein operativer Diskriminationsalgorithmus festgelegt ist, der eine für praktische Zwecke ausreichend vollständige Trennung der Teilbereiche ermöglicht. Dies ist dahingehend zu verstehen, daß eine für den jeweiligen medizinisch-analytischen Zweck ausreichend sichere Zuordnung einer Eingangsvariablen X zu einem der Teilbereiche der Kalibrationskurve $X=f^{-1}(C)$ möglich sein soll, wobei diese Zuordnung ohne zusätzlichen meßtechnischen Aufwand, insbesondere ohne zusätzliche Pipettierungsschritte (Probenverdünnung oder Erhöhung der Reagenzkonzentration) erreicht wird. Eine im mathematischen Sinne 100% richtige Trennung in die Teilbereiche ist hierzu vielfach nicht erforderlich. Insbesondere in der Nähe eines Maximums der Kalibrationskurve $X=f^{-1}(C)$ ist es vielfach klinisch nicht relevant, welchem der Teilbereiche ein Meßwert X zuzuordnen ist. Zum einen ist dort wegen des flachen Verlaufes der Kalibrationskurve $X=f^{-1}(C)$ der aus einer Fehlzuordnung resultierende Meßfehler der Konzentration C relativ gering. Zum zweiten liegt das Maximum meist im mittleren Konzentrationsbereich. Dieser mittlere Konzentrationsbereich entspricht den Normalwerten des zu bestimmenden Analyten und ist auch deswegen klinisch weniger relevant.

Überraschenderweise hat sich gezeigt, daß erfindungsgemäß mit einer geringen Anzahl von diskreten Meßwerten $S(t_1...t_n)$ eine sehr hohe Zuverlässigkeit der Zuordnung zu einem der Teilbereiche der Auswertekurve erreicht werden kann. Dies ist vor allem deshalb erstaunlich, weil in der Praxis jeder der Meßwerte $S(t_i)$ unvermeidlich mit einem statistischen Fehlerbeitrag insbesondere durch das Rauschen der Meßanordnung behaftet ist. Es konnte daher nicht erwartet werden, daß durch Selektion dieser Meßwerte und mit Hilfe von Ableitverfahren, die diese fehlerbehafteten Meßwerte in Beziehung zueinander setzen, ein Diskriminatorensatz abgeleitet werden kann, der unter Anwendung eines Verfahrens der multivariaten Statistik die Bestimmung eines die Teilbereiche eindeutig trennenden Grenzscores erlaubt. Ein wesentlicher Beitrag zu diesem Ergebnis ist bei dem erfindungsgemäßen Verfahren darauf zurückzuführen, daß es die selektierenden Diskriminatoren, bzw. die Ableitverfahren die zu diesen Diskriminatoren führen, selbst selektiert.

Die Erfindung wird im folgenden unter Bezugnahme auf die Figuren und nachfolgenden Beispiele näher erläutert; es zeigen:

Fig. 1 Eine graphische Darstellung mehrerer Kinetiken der Extinktion S für verschiedene Analytkonzentrationen bei einem Trübungstest,

Fig. 2 eine graphische Darstellung der nichtmonotonen Kalibrationskurve $X=f^{-1}(C)$ eines immunchemischen Trübungstests,

Fig. 3 eine graphische Darstellung der Verteilung der Scores bei einer Zuordnung auf Basis der vorliegenden Erfindung zu zwei Teilbereichen der Kalibrationskurve $X=f^{-1}(C)$,

Fig. 4 eine graphische Darstellung der Verteilung der Scores bei einer Zuordnung auf Basis der vorliegenden Erfindung zu drei Teilbereichen der Kalibrationskurve $X=f^{-1}(C)$.

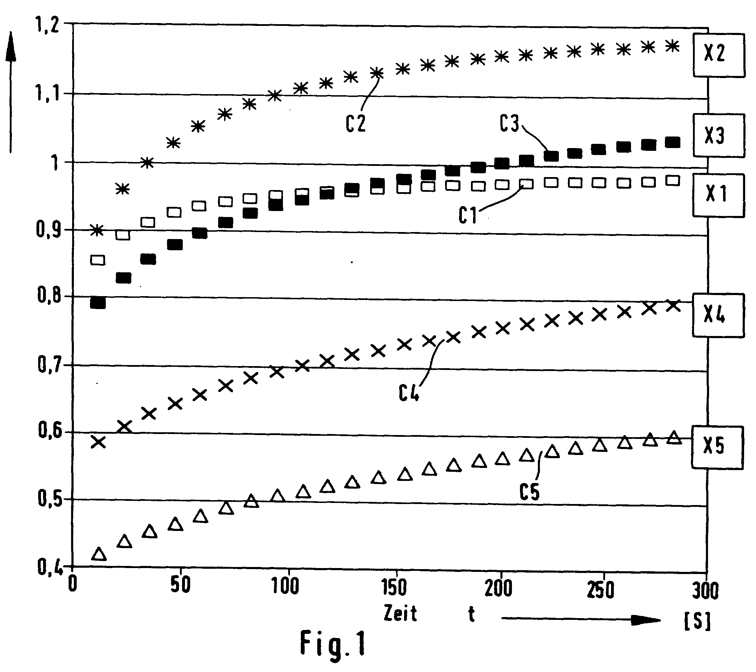

In Figur 1 ist der Verlauf der Extinktion S in willkürlichen Einheiten gegen die Zeit t in Sekunden für fünf verschiedene Konzentrationen $C_1....C_5$ aufgetragen, wobei $C_1$ der niedrigsten und $C_5$ der höchsten Konzentration entspricht.

Für beide einleitend beschriebenen Reaktionsmechanismen (heterogener Ein-Schritt-Sandwichtest, immunoturbidimetrischer Test) und auch für andere Testprinzipien, bei denen eine spezifische Bindungsreaktion stattfindet, die eine Brückenbildung oder Verknüpfung zweier Bindungspartner (z.B. Antikörpern) mittels eines dritten, multivalenten, mit den beiden anderen Bindungspartnern bindungsfähigen Bindungspartners einschließt, ist dieser prinzipielle Verlauf charakteristisch. Es handelt sich um Reaktionen, die mit vorgegebenen Anteilen der Reaktionspartner (welche außer der Analytkonzentration alle konstant sind) starten und nach einer Anstiegsphase asymptotisch in ein Meßwertplateau übergehen. Der zeitliche Verlauf der Reaktion sowie der Plateau-Wert werden beispielsweise bei dem genannten heterogenen Test durch die Anzahl der "Antigen-Brücken-Komplexe", beim turbidimetrischen Test durch die Anzahl der Antigen-Antikörper-Vernetzungen bestimmt.

Dadurch, daß sämtliche Faktoren außer der Analytkonzentration konstant gehalten werden, ist die Kinetik S(t) der Meßgröße S charakteristisch für die Konzentration C des Analyten. Üblicherweise wird aus der gemessenen Kinetik eine Eingangsvariable X für die Umrechnung in die Konzentration C mit Hilfe der Auswertekurve $C=f(X)$ abgeleitet. Die Kalibrationskurve $X=f^{-1}(C)$ wird mit Hilfe von Messungen an Kalibratoren (Standardproben, welche eine bekannte Konzentration des Analyten enthalten), die über den gesamten für die Analyse relevanten Konzentrationsbereich verteilt sind, bestimmt.

In der Praxis ist für die Erfindung nicht entscheidend, in welcher Weise die mit der Konzentration C korrelierende Eingangsvariable X aus dem gemessenen Verlauf der Kurve S(t) (d.h. der Kinetik der Reaktion) abgeleitet wird. Der Begriff der Eingangsvariablen X umfaßt jede Größe, die sich aus gemessenen Kinetiken bestimmen läßt und mit der Konzentration C korreliert. Wie dies im einzelnen geschieht, hängt von der jeweiligen Analyse und den Besonderheiten des Meßgerätes ab. Im Hinblick auf die Qualität der Analyse sollte sich die Eingangsvariable X in Abhängigkeit von der Konzentration möglichst stark ändern. Es muß sich nicht um eine physikalisch interpretierbare Größe (wie beispielsweise das Endpunkt-Meßsignal, welches der maximalen Trübung entspricht) handeln.

Als Eingangsvariable X sind bei Trübungsmessungen insbesondere, wie erläutert, der Endpunkt- oder Plateauwert der Trübung und die maximale Änderungsgeschwindigkeit $V_{max}$ gebräuchlich.

In Figur 1 entspricht $C_1$ der niedrigsten, $C_5$ der höchsten Konzentration. Man erkennt, daß beispielsweise der nach 300 sec. näherungsweise erreichte Endpunktwert $X_1...X_5$ sich nicht monoton mit der Konzentration ändert, sondern von $X_1$ nach $X_2$ ansteigt und dann bis $X_5$ abfällt.

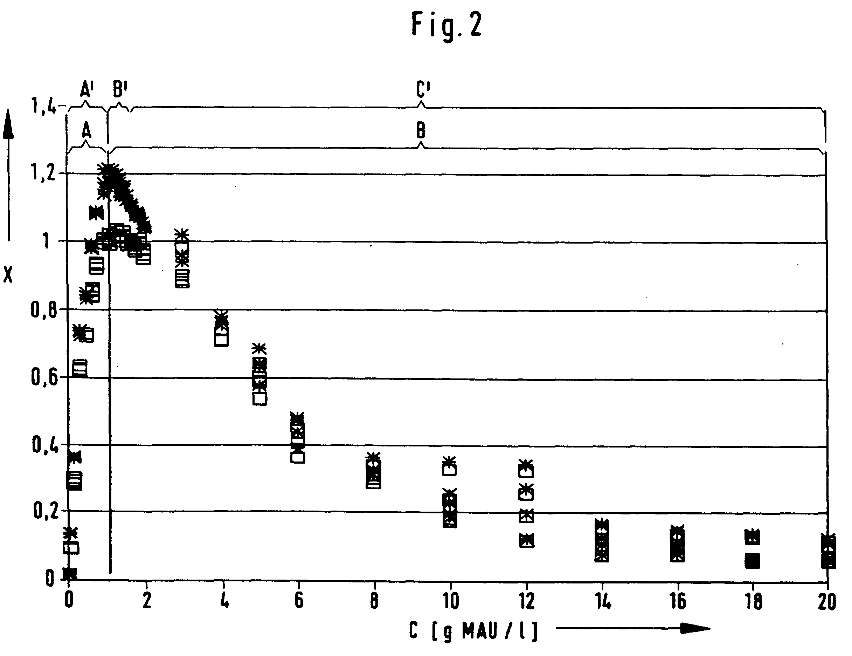

Hieraus resultiert der in Figur 2 dargestellte nichtmonotone Verlauf der Kalibrationskurve $X=f^{-1}(C)$ in Form einer Heidelberger-Kurve. Dargestellt sind Meßergebnisse mit zwei unterschiedlichen Reagenzchargen, wobei zu jeder Charge mehrere Messungen durchgeführt wurden. Dabei bezeichnen die Kreuze eine erste Charge und die Quadrate eine zweite Charge der Testreagenzien. Hierdurch wird die Meßwertstreuung sowohl von Messung zu Messung als auch von Charge zu Charge deutlich. X ist in willkürlichen Einheiten angegegeben. Die Konzentration C bezieht sich auf einen immunchemischen Test auf Albumin im Urin ("MAU"). Beispielsweise kann ein Meßwert X=0,8 einer Konzentration von 0,5 oder 3 g/l entsprechen, je nachdem, ob der C-Wert auf dem ansteigenden oder abfallenden Teilstück der Kalibrationskurve liegt. Die zum dargestellten Kurvenverlauf inverse Auswertekurve $C=f(X)$ ist zweideutig. Die eindeutige Angabe eines analytischen Ergebnisses setzt deshalb voraus, daß es gelingt, die aus S(t) abgeleiteten Eingangsvariablen X eindeutig einem der beiden Teilbereiche A oder B der Kalibrationskurve zuzuordnen und dadurch eine eindeutige Korrelation zu einer bestimmten Konzentration C zu erreichen. Zu diesem Zweck wird erfindungsgemäß folgendermaßen vorgegangen.

Zunächst wird ein Trainingslauf durchgeführt, der dazu dient, aus S(t)-Kinetiken, die an Kalibratoren gemessen wurden, einen auf den jeweiligen Analyse-Parameter, möglicherweise sogar auf eine bestimmte Reagenzcharge, spezifisch abgestimmten Diskriminationsalgorithmus, bestehend aus einem Diskriminatorensatz und einem Verfahren der multivariaten Statistik, zu entwickeln, der eine eindeutige Zuordnung gemessener Kinetiken S(t) zu einem der Teilbereiche der Auswertekurve erlaubt.

Zu diesem Zweck wird zunächst S(t) an einer Mehrzahl von Kalibratoren bekannter Konzentrationen bestimmt, wobei diese Konzentrationen in beiden Teilbereichen der Kalibrationskurve liegen. In der Praxis hat sich eine verhältnismäßig geringe Zahl solcher Kalibrator-Messungen, beispielsweise jeweils sechs Konzentrationen auf jedem Teilbereich A,B der Kalibrationskurve, als ausreichend erwiesen. Man erhält dann beispielsweise zwölf Kinetiken der in Figur 1 dargestellten Art, wobei zu jeder der Kinetiken die korrekte Konzentration C bekannt ist und somit auch festlegt, welchem der Teilbereiche A,B der Kalibrationskurve $X=f^{-1}(C)$ die gemessene Kinetik zuzuordnen ist.

Aus den gemessenen Kinetiken S(t) werden nach jeweils einem bestimmten definierten Verfahren, welches als Diskriminator-Generierungsverfahren bezeichnet wird, ein Satz von Diskriminatoren ermittelt. Im Rahmen der Erfindung wurden beispielsweise Extinktionswerte zu bestimmten Meßzeitpunkten, Steigungen, Krümmungen und Rauhigkeitswerte erfolgreich als Diskriminatoren verwendet. In dem bevorzugten Fall, daß S(t) nicht kontinuierlich, sondern zu diskreten Meßzeitpunkten $t_1...t_n$ bestimmt wird, lassen sich diese Diskriminatoren beispielsweise wie folgt berechnen.

Ein Extinktionswert $S_i$ zu einem Meßpunkt i (Meßzeitpunkt $t_i$) kann unmittelbar übernommen werden. Der Begriff des Diskriminator-Generierungsverfahrens umfaßt wie erwähnt auch Fälle, bei denen ein Meßwert S(t) für einen bestimmten Zeitpunkt selektiert und unverändert übernommen wird.

Eine Krümmung $K_i$ in einem Meßpunkt i läßt sich aus jeweils zwei benachbarten Meßpunkten $S_i$ und $S_{i+1}$ (gemessen zu den Zeitpunkten $t_i$ und $t_{i+1}$ wie folgt berechnen.

$$(1.1) \qquad K(i) = \frac{\alpha_{i+1} - \alpha_i}{T_i}$$

$$(1.2) \qquad \alpha_i = \text{atan} \frac{(S_{i+1} - S_i)}{t_{i+1} - t_i}$$

$$(1.3) \qquad T_i = \text{SQRT} \left[ (S_{i+1} - S_i)^2 + (t_{i+1} - t_i)^2 \right]$$

Darin bedeutet SQRT Quadratwurzel (square root).

Die Rauhigkeit ist ein statistischer Streuparameter, der sich als Diskriminator in bestimmten Bereichen bestimmter Analysen bewährt hat. Sie ist definiert durch

$$(2.1) \quad R = \frac{1}{n} \sum_{i=j+1}^{j+n} d_i$$

$$(2.2) \qquad d_i = \frac{(S_i - S_{i-1})}{S_i}$$

Außer den genannten sind zahlreiche andere Diskriminatoren im Rahmen der Erfindung verwendbar. In jedem Fall ist ein Diskriminator in diesem Sinne eine aus einer gemessenen Kinetik abgeleitete Variable, die durch ein definiertes Diskriminator-Generierungsverfahren eindeutig festgelegt ist, so daß sich aus einer bestimmten gemessenen Kinetik unter Anwendung dieses Diskriminator-Generierungsverfahrens stets der gleiche Wert des Diskriminators ergibt.

Prinzipiell können zusätzlich andere, nicht aus den Kinetiken S(t) abgeleitete Größen als Hilfsdiskriminatoren verwendet werden, wie beispielsweise Geräteinstellungen oder Meßwerte über Umweltbedingungen.

Jeder der Diskriminatoren ist einem Konzentrationswert $C_j$ und einem Meßzeitpunkt $t_i$ zugeordnet. Der Diskriminatorensatz ist demzufolge von den beiden unabhängigen Variablen C und t abhängig. Eine derartige von mehreren Variablen abhängige mehrdimensionale Wertematrix kann mit bekannten Verfahren der multivariaten Statistik aus dem mehrdimensionalen Raum in einen eindimensionalen Raum überführt werden. Im Falle der vorliegenden Erfindung wird ein Verfahren der multivariaten Statistik dazu benutzt, aus den Diskriminatoren einer Kinetik (d.h. aus den Diskriminatoren, die aus dem an einem Kalibrator gemessenen Verlauf von S(t) bestimmt wurden) Zahlenwerte, sogenannte Scores zu berechnen. Da bekannt ist, in welchem Teilbereich der Heidelberger-Kurve die jeweilige Konzentration des Kalibrators liegt, ist jeder der berechneten Scores einem der Teilbereiche A oder B der Heidelberger-Kurve zugeordnet.

Nähere Informationen über Verfahren der multivariaten Statistik können der einschlägigen Literatur entnommen werden. Verwiesen sei beispielsweise auf folgende Publikationen:

L. Fahrmeir u. A. Hamerle, "Multivariate statistische Verfahren", Walter de Gruyter, New York 1984. Darin wird u.a. die Diskriminanzanalyse und die Schätzung von Verteilungen eines Merkmalsvektors mittels einer Lernstichprobe beschrieben. SAS/STAT-User's Guide, Release 6.03, SAS-Institute Inc.

Als nächstes wird geprüft, ob die berechneten Scores aller Kalibratoren sich eindeutig in zwei Teilmengen teilen lassen, dergestalt, daß sämtliche Scores, die einem Konzentrationswert im ansteigenden Teil A der Heidelberger-Kurve entsprechen auf einer Seite des Grenzwertes liegen (beispielsweise kleiner als dieser Grenzwert sind), während die Scores, die einem Konzentrationswert auf dem abfallenden Teilbereich B der Heidelberger-Kurve entsprechen, auf der anderen Seite dieses Grenzwertes liegen (also beispielsweise größer als der Grenzwert sind). Ein Grenzwert, der eine solche Trennung ermöglicht, wird als Grenzscore bezeichnet. Der Grenzscore wird im Regelfall mit dem gleichen Verfahren der multivariaten Statistik berechnet, mit dem die Scores aus den Kinetiken berechnet werden.

In der Regel wird bei der vorstehenden Prüfung festgestellt werden, daß eine für praktische Zwecke ausreichende Trennung durch einen Grenzscore auf Basis des zunächst erprobten Diskriminatorensatzes nicht möglich ist. Ein wesentliches Element der vorliegenden Erfindung ist darin zu sehen, daß in der Trainingsphase der operative Diskriminatorensatz durch Iteration der vorgenannten Schritte ermittelt wird. Die Variation der Diskriminator-Generierungsverfahren, die zu den Diskriminatoren führen, kann dabei völlig stochastisch geschehen, d.h. das System enthält eine Auswahl von Diskriminator-Generierungsverfahren, die nacheinander durchpermutiert werden, bis das gewünschte Ergebnis (unter Berücksichtigung der medizinisch-analytischen Erfordernisse ausreichende Trennung der Scores durch einen Grenzscore) erreicht wird. Selbstverständlich kann in dieser Trainingsphase auch auf Basis vorgegebener Erfahrungswerte gezielt eingegriffen werden.

Insgesamt besteht der Trainingslauf aus einer Mehrzahl von immer wieder wiederholten Abschnitten, in denen jeweils ein Diskriminationsalgorithmus abläuft, der aus einem Satz von Diskriminator-Generierungsverfahren zur Bestimmung der Diskriminatoren und aus einem definierten Verfahren der multivariaten Statistik besteht, welches eine Abbildung des mehrdimensionalen Raumes der Diskriminatoren auf die Eindimensionalität der Scores ermöglicht.

Dabei können außer den Diskriminator-Generierungsverfahren auch die verwendeten Verfahren der multivariaten Statistik variiert werden. Auch dies kann grundsätzlich ohne menschliches Eingreifen erfolgen, wobei in dem System unterschiedliche Verfahren der multivariaten Statistik implementiert sind, welche jeweils mit dem gleichen Diskriminatorensatz oder auch mit unterschiedlichen Diskriminatorensätzen in dem Trainingslauf erprobt werden. Die Erprobung der Erfindung hat gezeigt, daß in der Praxis Erfahrungswerte gewonnen werden können, wonach für bestimmte Analysen bestimmte Verfahren der multivariaten Statistik geeignet sind. In diesen Fällen wird man zweckmäßigerweise für die Bestimmung eines bestimmten Analyten dem System dieses bewährte Verfahren der multivariaten Statistik vorgeben und in dem Trainingslauf nur noch die Diskriminator-Generierungsverfahren variieren. Dies ist jedoch nicht zwingend. Es sind auch Konstellationen möglich, bei denen es vorteilhaft ist, einen bestimmten Diskriminatoren-Satz festzuhalten und in dem Trainingslauf in Kombination mit unterschiedlichen Verfahren der multivariaten Statistik zu erproben.

Ein Diskriminationsalgorithmus, der die gewünschte Trennung der Scores in zwei den Teilbereichen der Auswertekurve zugeordneten Teilmengen mit (im erläuterten Sinn) für praktische Zwecke ausreichender Zuverlässigkeit erlaubt, wird als operativer Diskriminationsalgorithmus bezeichnet.

Je nach den Erfordernissen des Einzelfalles kann in der Praxis die Bestimmung des operativen Diskriminationsalgorithmus zentral bei dem Hersteller des für die analytische Bestimmung verwendeten Reagenzien-Kits oder dezentral in dem jeweiligen automatischen Analysegerät erfolgen.

a) Die zentrale Bestimmung durch den Hersteller hat den Vorteil, daß eine besonders leistungsfähige Computeranlage eingesetzt werden kann. Wenn der optimierte Diskriminationsalgorithmus von der Herstellungscharge des Reagenzienkits unabhängig ist, genügt eine einmalige Übermittlung der Information an das Analysegerät, beispielsweise mit Hilfe einer Diskette. Wenn sich aber verschiedene Herstellungschargen der Reagenzien derartig unterscheiden, daß der gleiche optimierte Diskriminationsalgorithmus nicht für verschiedene Chargen verwendet werden kann, ist es zweckmäßig, daß der optimierte Diskriminationsalgorithmus separat für jede Charge ermittelt und mit der Packung der Testkits dem klinischen Labor auf einem geeigneten Datenträger zur Verfügung gestellt wird. Es ist dabei nicht erforderlich, auf dem Analysegerät Kalibratoren zu vermessen, die den gesamten relevanten Meßbereich der analytischen Bestimmung abdecken.

b) Auf dem Gerät erfolgt die Bestimmung eines optimierten Diskriminatorensatzes parallel mit der Bestimmung der Kalibrationskurve. Die Messung der Kinetiken S(t) unterscheidet sich dabei im Prinzip nicht von üblichen Kalibrationskurvenbestimmungen. Es ist jedoch erforderlich, daß die Kalibratoren den Meßbereich weitgehend abdecken und eine ausreichende Zahl von Kalibratorkonzentrationen jeweils in beiden Teilbereichen der Heidelberger-Kurve liegt. Bei Geräten, die diese Bedingungen erfüllen, d.h. die die Bestimmung der vollständigen nichtlinearen Kalibrationskurve $X=f^{-1}(C)$ unterstützen, ist praktisch kein zusätzlicher konstruktiver oder meßtechnischer Aufwand erforderlich. Aus den im Rahmen der Kalibration ohnehin bestimmten Kinetiken S(t) kann der optimierte Kalibrationsalgorithmus mit Hilfe des in solchen Geräten üblicherweise ohnehin vorhandenen Computers ermittelt werden.

Selbstverständlich sind Mischformen aus den beiden vorgenannten Varianten a) und b) möglich.

In einem Analyselauf wird der optimierte Diskriminationsalgorithmus folgendermaßen verwendet.

Die Kinetik S(t) wird an einer zu analysierenden Probe bestimmt. Sofern die Messung zu diskreten Meßzeitpunkten $t_i$ erfolgt, sollten die Meßzeitpunkte, jeweils gerechnet vom Reaktionsstart, bei dem Analyselauf mit den beim Trainingslauf verwendeten Meßzeitpunkten übereinstimmen.

Auf die Kinetiken wird der operative Diskriminationsalgorithmus angewandt, d.h. mit Hilfe der gleichen Diskriminator-Generierungsverfahren werden Diskriminatoren generiert und aus diesen wird mit Hilfe des gleichen Verfahrens der multivariaten Statistik ein Analysescore berechnet. Durch einfachen Vergleich des Analysescores mit dem Grenzscore kann dann die Zuordnung der aus der Kinetik S(t) abgeleiteten Eingangsvariablen X zu einem der Teilbereiche der Kalibrationskurve $X=f^{-1}(C)$ erfolgen.

Unter den Verfahren der multivariaten Statistik sind die Verfahren der Diskriminanzanalyse besonders bevorzugt.

Allgemein wird die Diskriminanzanalyse dazu benutzt, Objekte aufgrund ihrer p-Merkmale (p-dimensionaler Merkmal-Vektor) möglichst eindeutig Gruppen zuzuordnen. Die Gruppenzugehörigkeit jedes Einzelobjektes liegt fest und ist prüfbar. Ausgehend von einem Set von Objekten mit bekannter Gruppenzugehörigkeit, hier der Kinetiken, wird in einem p-dimensionalen Raum diejenige Projektion auf einen eindimensionalen Raum ermittelt, die die bestmögliche Trennung der Gruppen ergibt.

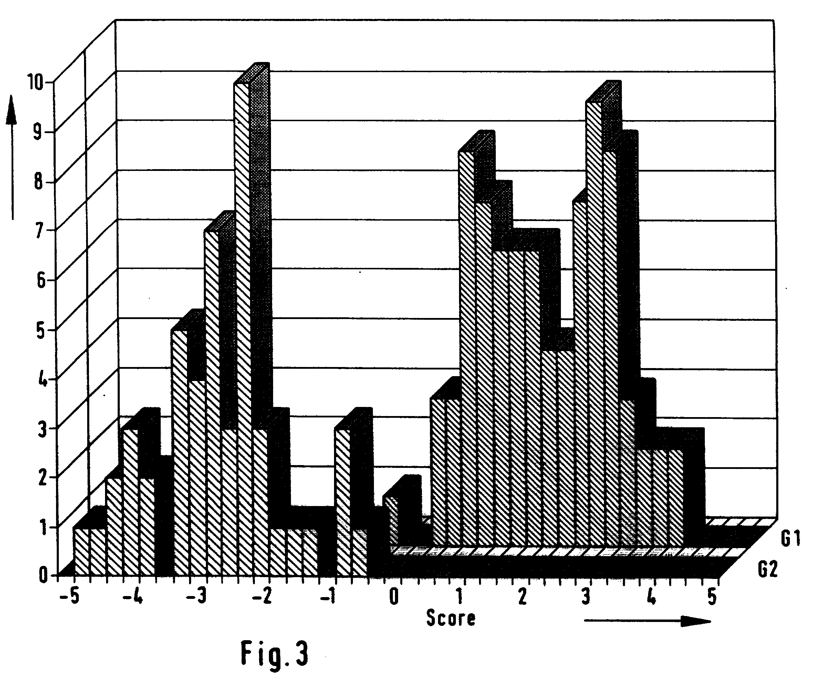

Figur 3 zeigt beispielshaft eine eindimensionale Häufigkeitsverteilung für die Scores von zwei Gruppen G1 und G2 von Kinetiken mit einer Diskriminierung, die besser ist als 95%. Die Diskriminierung der Kinetiken ist hier also nicht zu 100% gelungen. Man erkennt, daß ein kleiner Teil der in der Figur rechts dargestellten Gruppe G1 einen Score im negativen Wertebereich der Abszisse hat. Obwohl der Diskriminationsalgorithmus in diesem Fall keine im mathematischen Sinne vollständige Trennung der Kinetiken erlaubt, kann ein solches Ergebnis für praktische Zwecke vielfach vollständig ausreichend sein, soweit die wenigen Fehlzuordnungen aus den weiter oben erläuterten Gründen für das medizinisch-analytische Ergebnis ohne praktische Bedeutung sind.

Für die Diskriminierung wurden im Zusammenhang der vorliegenden Erfindung unterschiedliche Diskriminanzanalyse-Verfahren erfolgreich eingesetzt. Dies sind die klassische lineare und quadratische Diskriminanzanalyse sowie nichtparametrische Methoden, unter anderem Kernschätzer und nächste Nachbar-Methode. Bei den Kernschätzern erwiesen sich die Kerntypen Uniform, normal und Epanechnikow als geeignet.

Die mathematische Literatur beschreibt ausführlich solche Methoden. Verwiesen sei beispielsweise auf die erwähnten Publikationen über Verfahren der multivariaten Statistik und:

B.W. Silverman, Density Estimation for Statistics and Data Analysis, Chapman & Hall, London, New York 1986.

Für die Schätzung der Fehlerrate haben sich die Resubstitutions- und die Crossvalidierungs-Methode als geeignet erwiesen. Bei der Resubstitution wird für den betrachteten Satz von Kinetiken die Rate der Fehlzuordnungen bestimmt. Bei der Crossvalidationsmethode wird die Analyse mit (n-1) Kinetiken durchgeführt und überprüft, ob die Zuordnung der n-ten Kinetik richtig erfolgt ist. Dann wird durchpermutiert und anschließend die Fehlerrate ermittelt.

Beispiele

Beispiel 1:

Um die Konzentration von Albumin in Urin (zur Diagnose der Mikroalbuminurie) zu bestimmen, wurde ein klassischer turbidimetrischer Trübungstest durchgeführt (mit Reagenzien, die unter dem Namen "Tinaquant™, Mikroalbuminurie", Hersteller: Boehringer Mannheim GmbH, Mannheim, Bundesrepublik Deutschland, kommerziell erhältlich sind). Die Analysen erstreckten sich auf einen Konzentrationsbereich von 0 bis 10.000 mg MAU/l. Dabei wurden zwei unterschiedliche Reagenzchargen verwendet, die sich insbesondere durch die Herstellungscharge der Antikörper unterschieden.

Zur Messung wurde ein Analysegerät Hitachi 717™ verwendet, bei welchem die photometrische Messung in einem Takt von 12 Sekunden, also mit einer Frequenz von fünf Messungen pro Minute erfolgt. Für jede Probe erfolgten dabei innerhalb einer Kinetik die erste Messung nach zwölf Sekunden und danach weitere Messungen mit der genannten Meßfrequenz, wobei das Gerät an jedem Meßpunkt eine Dreifachmessung durchführt und den mittleren Meßwert (Median) für die Weiterverarbeitung verwendet.

Als Eingangsvariable X wurde die Differenz zwischen den Extinktionen nach 300 Sekunden und nach 12 Sekunden verwendet: X = S(300 sec) - S(12 sec).

Auf diese Weise wurden in einer ersten Meßreihe mit jeder Antikörpercharge je fünf Proben mit zwölf vorbekannten Konzentrationen (0;250;500;1000;1500;2000;3000;4000;5000;6000; 8000;10000 mg MAU/dl) vermessen. In der zweiten Meßreihe wurden Proben mit 42 vorbekannten Konzentrationen (0;100;200;...;1000;1250;1500;....;4750;5000;6000;...; 10000 mg MAU/l) jeweils dreifach vermessen. Es wurden somit in der ersten Meßreihe für zwölf C-Werte jeweils fünf X-Werte und in der zweiten Meßreihe für 42 C-Werte jeweils drei C-Werte bestimmt, die sich in der in Figur 2 dargestellten Weise als nichtmonotone Heidelberger-Kurve darstellen lassen.

Auf Basis der gemessenen Kinetiken wurden in der beschriebenen Art und Weise in einem Trainings lauf operative Diskriminationsalgorithmen ermittelt. Als Diskriminatoren wurden dabei fünf Krümmungen für die Meßzeitpunktpaare $t_1/t_2$, $t_2/t_3$, $t_3/t_4$, $t_4/t_5$ und $t_5/t_6$ (jeweils berechnet nach den Formeln 1.1 bis 1.3) sowie der fünfte Extinktionswert nach der Pipettierung des zweiten Reagenz (d.h. Start der Trübungsreaktion) ermittelt, aus denen mit dem Statistikpaket SAS (Hersteller: SAS Institute Inc., Cary, N.C., USA), einem Verfahren der kanonischen Diskriminanzanalyse, Scores und ein Grenzscore berechnet wurden.

Um die Qualität der Zuordnung der gemessenen Ergebnisse zu prüfen, wurde zunächst mit den Kinetiken der Charge 1 der Trainingslauf durchgeführt, d.h. die Koeffizienten des Diskriminanz-Analyseverfahrens und der Grenzscore wurden in einem Trainingslauf der beschriebenen Art bestimmt. Mit Hilfe der so erhaltenen Koeffizienten wurden die Scores aller Reaktionskinetiken, die mit Hilfe der Reagenzcharge 2 gemessen worden waren, berechnet. Aus ihrer Relation zu dem Grenzscore wurde die Zuordnung der jeweiligen Reaktionskinetik zum Bereich A bzw. zum Bereich B der Heidelberger-Kurve bestimmt. Das gleiche Verfahren wurde umgekehrt wiederholt, wobei zum Trainieren die an Charge 2 gemessenen Kinetiken verwendet und anschließend die Kinetiken aus Charge 1 analysiert wurden.

In beiden Fällen gelang die Diskriminierung zu 100%.

Das gleiche vorteilhafte Ergebnis wurde auch erhalten, wenn ausgesuchte Untermengen der Meßergebnisse zu beiden Chargen als Trainingsset für den Trainingslauf verwendet wurden. Insbesondere wurde nur mit den 12 Konzentrationen der ersten Meßreihe trainiert und die 42 Konzentrationen der zweiten Meßreihe wurden richtig zugeordnet.

Beispiel 2:

Mit Hilfe eines Latex-verstärkten immunoturbidimetrischen Testverfahrens ("Tina-quant™, Ferritin", Hersteller: Boehringer Mannheim GmbH) wurde Ferritin in Serumproben bekannter Konzentration bestimmt. Dabei wurden mit zwei verschiedenen Reagenzchargen jeweils 22 verschiedene Konzentrationen im Bereich zwischen 0 und 6000 ng Ferritin/ml vermessen, wobei bei der einen Charge eine Sechsfach-Bestimmung, bei der zweiten Charge eine Zehnfach-Bestimmung durchgeführt wurde. Auch in diesem Fall wurden mit Hilfe eines Trainingslaufs der beschriebenen Art die Diskriminatoren und ein Verfahren der multivariaten Statistik so weit optimiert, bis ein operativer Diskriminationsalgorithmus gefunden war. Als Diskriminatoren erwiesen sich beispielsweise folgende Größen als geeignet: Die zweite bis vierte Krümmung ($t_2/t_3$;$t_3/t_4$;$t_4/t_5$) sowie die fünfte und 25. Extinktion (jeweils nach der zweiten Reagenzpipettierung bei einer Meßfrequenz von fünf Messungen pro Minute). Das ausgewählte Verfahren war ein nicht-parametrisches Diskriminanzverfahren des erwähnten Statistik-Programmpaketes SAS.

Als Trainingsset für den Trainingslauf wurden in einem ersten Versuch von sämtlichen 22 Konzentrationen drei der sechs Kinetiken für den Trainingslauf verwendet. In einem zweiten Versuch wurden von den 22 Konzentrationen nur 11 für den Trainings lauf verwendet, welche so ausgewählt waren, daß sie den gesamten Konzentrationsbereich abdecken.

Die jeweils verbliebenen Kinetiken wurden als Proben für den Analyselauf (Anwendungsset) verwendet. Mit den in dem Trainingslauf ermittelten Koeffizienten des DKA-Verfahrens wurden Scores berechnet und aus der Relation zum Grenzscore die Zuordnung der Reaktionskinetik zu den Bereichen A und B der Heidelberger-Kurve bestimmt. Es wurde eine 98 % richtige Zuordnung erreicht. Sämtliche Fehlzuordnungen waren jedoch klinisch nicht relevant im obigen Sinne. Bei Wertung nur der klinisch relevanten Fehlzuordnungen wurde wiederum eine 100 % richtige Zuordnung erreicht.

Beispiel 3:

Dieses Beispiel entsprach experimentell Beispiel 1. Es wurden die gleichen Diskriminatoren und Verfahren der Diskriminanzanalyse verwendet. Die Kalibrationskurve wurde jedoch in diesem Fall in drei Teilbereiche (A',B',C' in Fig. 2) geteilt, nämlich einen ersten Teilbereich von 0 bis 1,1 g MAU/ml, einen zweiten Bereich von 1,1 bis 1,5 g MAU/ml und einen dritten Teilbereich von 1,5 g MAU/ml bis 7 g MAU/ml. Der mittlere Konzentrationsbereich war dabei so gewählt, daß er jeweils einen relativ kleinen Teilbereich links und rechts des Maximums der Heidelberger-Kurve erfaßt.

Wenn die Kalibrationskurve in mehr als zwei Teilbereiche geteilt ist, erhöht sich die Anzahl der erforderlichen Grenzscores. Bei drei Teilbereichen sind beispielsweise zwei Grenzscores erforderlich, um den mittleren Bereich jeweils zu den beiden anderen Bereichen abzugrenzen.

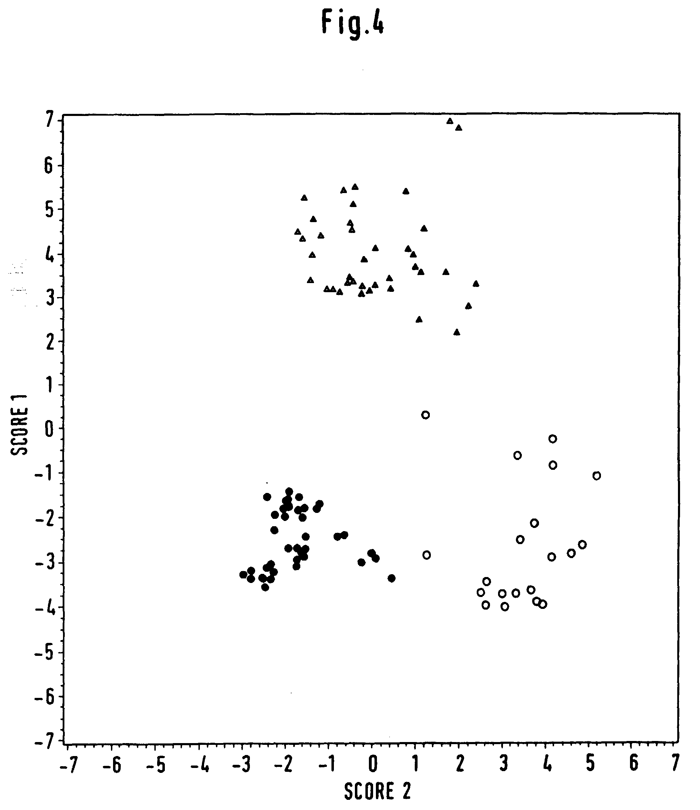

In diesem Fall wurde keine kanonische Diskriminanzanalyse, sondern ein nicht-parametrisches Verfahren mit einer Gausskurve als Kernfunktion (Statistik-Programmpaket SAS) verwendet. Es wurde eine 100 % richtige Zuordnung der Extinktionswerte zu den drei Teilbereichen erreicht. Figur 4 zeigt eine graphische Darstellung der Ergebnisse, wobei die Scores für die einzelnen gemessenen Konzentrationen in zwei Dimensionen aufgetragen sind. Dabei sind als ausgefüllte Punkte die Scores zu dem ersten Konzentrationsbereich A', als Kreise die Scores zu dem zweiten Konzentrationsbereich B' und als Dreiecke die Scores zu dem dritten Konzentrationsbereich C' dargestellt.

**Patentansprüche**

1.   Verfahren zur analytischen Bestimmung der Konzentration eines Bestandteiles einer medizinischen Probe,

bei dem eine Reaktion der Probe mit Reagenzien zu einer zeitabhängigen Änderung S(t) einer Meßgröße S führt und

die Konzentration C gemäß einer Auswertekurve C(X) mit einer aus S(t) abgeleiteten Eingangsvariablen X korreliert,

wobei die Kalibrationskurve X=f$^{-1}$(C) nicht monoton ist, so daß der gleiche Wert der Eingangsvariablen X auf mindestens zwei Teilbereichen der Kalibrationskurve unterschiedlichen Werten der Konzentration C entspricht und die Auswertekurve mindestens für einen Teil der möglichen X-Werte mehrdeutig ist,

bei welchem folgende Schritte durchgeführt werden, um eine Eingangsvariable X einem der Teilbereiche zuzuordnen und dadurch eine eindeutige Korrelation zu einer bestimmten Konzentration C zu erreichen:

a. In einem Trainingslauf wird mindestens einmal ein Diskriminationsalgorithmus mit nachfolgenden Schritten durchgeführt

a.a S(t) wird an mehreren Kalibratoren bekannter Konzentrationen $C_j=C_1...$ $C_m$ bestimmt, wobei die Konzentrationen $C_j=C_1...C_m$ in den zwei Teilbereichen der Kalibrationskurve X=f$^{-1}$(C) liegen;

a.b aus den Messungen von S(t) wird nach vorbestimmten Diskriminator-Generierungsverfahren ein Diskriminatorensatz generiert;

a.c aus dem Diskriminatorensatz wird mit einem Verfahren der multivariaten Statistik je ein Score für jede der Konzentrationen $C_j=C_1...C_m$ der Kalibratoren berechnet;

a.d es wird geprüft, ob sich die Scores für die Konzentrationen $C_j=C_1...c_m$ der Kalibratoren mittels eines Grenzscores in getrennte Teilmengen teilen lassen, bei denen die Konzentrationen den Teilbereichen der Kalibrationskurve richtig zugeordnet sind;

b. In dem Trainingslauf wird der die Schritte a.a bis a.d umfassende Diskriminationsalgorithmus mit verschiedenen, nach unterschiedlichen Generierungsverfahren aus den Messungen von S(t) generierten Diskriminatorensätzen und/oder unterschiedlichen Verfahren der multivariaten Statistik wiederholt, um einen operativen Diskriminationsalgorithmus festzulegen, der in dem Schritt a.d eine für praktische Zwecke ausreichend vollständige Trennung der Teilbereiche ermöglicht;

c. in einem Analyselauf wird:

c.a S(t) an einer zu analysierenden Probe bestimmt;

c.b daraus nach dem operativen Diskriminationsalgorithmus ein Analysescore berechnet;

c.c der Analysescore mit dem Grenzscore verglichen und

c.d die Eingangsvariable X und daraus die Konzentration C bestimmt, wobei der Wert X einer der Teilbereiche der Kalibrationskurve $X=f^{-1}(C)$ durch Vergleich des Analysescore mit dem Grenzscore zugeordnet wird.

2. Verfahren nach Anspruch 1, bei welchem S(t) in den Schritten a.a und c.a diskontinuierlich zu diskreten Meßzeitpunkten $t_1...t_n$ bestimmt wird.

3. Verfahren nach Anspruch 2, bei welchem die Meßzeitpunkte in dem Schritt c.a mit den Meßzeitpunkten in Schritt a.a, jeweils bezogen auf den Startzeitpunkt der Reaktion, übereinstimmen.

4. Verfahren nach Anspruch 2 oder 3, bei welchem die Meßzeitpunkte $t_1...t_n$ einen zeitlichen Abstand von mindestens 0,5 sec haben.

5. Verfahren nach einem der vorhergehenden Ansprüche, bei welchem der Diskriminatorensatz die Steigung, die Krümmung und/oder die Rauhigkeit der S(t)-Kurve zu bestimmten Meßzeitpunkten einschließt.

6. Verfahren nach einem der vorhergehenden Ansprüche, bei welchem das Verfahren der multivariaten Statistik ein Verfahren der Diskriminanzanalyse ist.

7. Verfahren nach einem der vorhergehenden Ansprüche, bei welchem die Reaktion eine spezifische Bindungsreaktion zweier bioaffiner Bindungspartner einschließt.

8. Verfahren nach Anspruch 7, bei welchem die Meßgröße S das Resultat einer Trübungsmessung ist.

9. Verfahren nach Anspruch 8, bei welchem einer der bioaffinen Bindungspartner ein multifunktionaler Antikörper-Träger zur Verstärkung einer Immunpräzipitation ist.

10. Verfahren nach einem der vorhergehenden Ansprüche, bei welchem die Kalibrationskurve in mehr als zwei Teilbereiche (A',B',C') geteilt ist, der Trainingslauf für jede Zweierkombination der Teilbereiche (A',B';A',C';B',C') gesondert durchgeführt wird, um jeweils einen operativen Diskriminationsalgorithmus festzulegen und in dem Analyselauf jeder der operativen Diskriminationsalgorithmen auf jede gemäß dem Schritt c.a. gemessene zeitabhängige Änderung S(t) angewandt wird, um die gemessenen Eingangsvariablen X den Teilbereichen A',B',C' zuzuordnen.

**Claims**

1. Method for the analytical determination of the concentration of a component of a medical sample,

in which a reaction of the sample with reagents leads to a time-dependent change S(t) of a measured quantity S and

the concentration C correlates according to an evaluation curve C(X) with an input variable X derived from S(t),

in which the calibration curve $X=f^{-1}(C)$ is not monotonous, so that the same value of the input variable X corresponds on at least two sub-sections of the calibration curve to different values of the concentration C and the evaluation curve is ambiguous at least for a portion of the possible X values,

in which the following steps are performed in order to assign an input variable X to one of the sub-sections and thereby to achieve an unambiguous correlation to a particular concentration C:

a. In a training run a discrimination algorithm is performed at least once with the following steps

a.a S(t) is determined on several calibrators of known concentrations $C_j=C_1...C_m$, the concentrations $C_j=C_1...C_m$ lying in the two sub-sections of the calibration curve $X=f^{-1}(C)$;

a.b from the measurements of (S)t a discriminator set is generated according to a pre-determined discriminator generation method;

a.c from the discriminator set there is calculated, by a multivariate statistical technique, a score for each of the concentrations $C_j=C_1...C_m$ of the calibrators;

a.d it is checked whether the scores for the concentrations $C_j=C_l...C_m$ of the calibrators can be divided by means of a boundary score into separate subsets, in which the concentrations are correctly assigned to the sub-sections of the calibration curve;

b. in the training run the discrimination algorithm embracing the steps a.a to a.d is repeated with different discriminator sets generated from the measurements of S(t) by various generation methods and/or with different multivariate statistical techniques, in order to establish an operative discrimination algorithm which makes possible in the step a.d a separation of the sub-sections which is sufficiently precise for practical purposes;

c. in an analysis run:

c.a S(t) is determined from a sample to be analysed;

c.b from the latter an analysis score is calculated according to the operative discrimination algorithm;

c.c the analysis score is compared with the boundary score and

c.d the input variable X and from the latter the concentration C is determined, the value X being assigned to one of the sub-sections of the calibration curve $X=f^{-1}(C)$ by comparison of the analysis score with the boundary score.

2. Method according to claim 1, in which S(t) is determined in the steps a.a and c.a discontinuously at discrete measuring times $t_1...t_n$.

3. Method according to claim 2, in which the measuring times in the step c.a coincide with the measuring times in step a.a, referred in each case to the starting time of the reaction.

4. Method according to claim 2 or 3, in which the measuring times $t_1...t_n$ are measured at a time interval of at least 0.5 sec.

5. Method according to any one of the preceding claims, in which the discriminator set includes the slope, the -curvature and/or the roughness of the S(t) curve at particular measuring times.

6. Method according to any one of the preceding claims, in which the multivariate statistical technique is a discriminant analysis technique.

7. Method according to any one of the preceding claims, in which the reaction includes a specific binding reaction of two binding partners exhibiting biological affinity.

8. Method according to claim 7, in which the measured quantity S is the result of a turbidity measurement.

9. Method according to claim 8, in which one of the binding partners exhibiting biological affinity is a polyfunctional antibody carrier for the amplification of an immune precipitation.

10. Method according to any one of the preceding claims, in which the calibration curve is divided into more than two sub-sections (A',B',C'), the training run is performed separately for each paired combination of the sub-sections (A',B';A',C';B',C') in order to establish in each case an operative discrimination algorithm, and in the analysis run each of the operative discrimination algorithms is applied to each time-dependent change S(t) measured according to the step c.a, in order to assign the measured input variables X to the sub-sections A',B',C'.

## Revendications

1. Procédé pour la détermination analytique de la concentration d'un composant d'un échantillon médicinal,

   dans lequel une réaction de l'échantillon avec des réactifs entraîne une variation en fonction du temps S(t) d'une grandeur mesurée S et la concentration C, selon une courbe d'évaluation C(X), est corrélée avec une variable d'entrée X obtenue à partir de S(t),
   la courbe de calibration $X=f^{-1}(C)$ n'étant pas monotone de sorte que la même valeur de la variable d'entrée X correspond à des valeurs différentes de la concentration C sur au moins deux régions partielles de la courbe de calibration, et la courbe d'évaluation est équivoque pour au moins une partie des possibles valeurs X,
   dans lequel on effectue les étapes suivantes pour associer une variable d'entrée X à l'une des régions partielles et obtenir ainsi une corrélation univoque avec une concentration C déterminée :

   a. Dans une séquence d'entraînement, on fait fonctionner au moins une fois un algorithme de discrimination comprenant les étapes suivantes :

   a.a on détermine S(t) à l'aide de plusieurs calibrateurs de concentrations connues $c_j=c_1 \ldots c_m$, les concentrations $c_j=c_1 \ldots c_m$ étant situées dans les deux régions partielles de la courbe de calibration $X=f^{-1}(C)$;
   a.b à partir des mesures de S(t), selon des méthodes de génération de discriminateurs prédéfinies, on génère un groupe de discriminateurs;
   a.c à partir du groupe de discriminateurs, à l'aide d'une méthode de la statistique multivariée, on calcule un résultat pour chacune des concentrations $c_j=c_1 \ldots c_m$ des calibrateurs ;
   a.d on vérifie si à l'aide d'un résultat limite on peut diviser les résultats pour les concentrations $c_j=c_1 \ldots c_m$ des calibrateurs en sous-ensembles séparés, dans lesquels les concentrations sont associées correctement aux régions partielles de la courbe de calibration ;

   b. Dans la séquence d'entraînement, on répète l'algorithme de discrimination comprenant les étapes a.a - a.d en utilisant différents groupes de discriminateurs générés selon différentes méthodes de génération de discriminateurs à partir des mesures de S(t) et/ou différentes méthodes de la statistique multivariée, pour définir un algorithme de discrimination opérationnel permettant d'obtenir dans l'étape a.d une séparation des régions partielles, qui soit suffisamment complète pour des fins pratiques ;
   c. Dans une séquence d'analyse:

   c.a on détermine S(t) à l'aide d'un échantillon a analyser;
   c.b à partir de ceci, on calcule un résultat d'analyse selon l'algorithme de discrimination opérationnel ;
   c.c on compare le résultat d'analyse avec le résultat limite et
   c.d on détermine la variable d'entrée X et à partir de celle-ci la concentration C, la valeur X étant associée à l'une des régions partielles de la courbe de calibration $X=f^{-1}(C)$ par comparaison du résultat d'analyse avec le résultat limite.

2. Procédé selon la revendication 1, dans lequel on détermine S(t) de façon discontinue dans les étapes a.a et c.a, à des instants de mesure discrets $t_1 \ldots t_n$.

3. Procédé selon la revendication 2, dans lequel les instants de mesure dans l'étape c.a correspondent aux instants de mesure dans l'étape a.a, rapportés à chaque fois à l'instant de démarrage de la réaction.

4. Procédé selon la revendication 2 ou 3, dans lequel les instants de mesure $t_1 \ldots t_n$ sont espacés d'au moins 0,5 sec.

5. Procédé selon l'une quelconque des revendications précédentes, dans lequel le groupe de discriminateurs comprend la pente, la courbure et/ou la rugosité de la courbe S(t) à des instants de mesure définis.

6. Procédé selon l'une quelconque des revendications précédentes, dans lequel la méthode de la statistique multivariée est une méthode de l'analyse discriminante.

7. Procédé selon l'une quelconque des revendications précédentes, dans lequel la réaction comprend une réaction de liaison spécifique de deux partenaires de liaison bioaffines.

8. Procédé selon la revendication 7, dans lequel la grandeur mesurée S est le résultat d'une turbidimétrie.

9. Procédé selon la revendication 8, dans lequel l'un des partenaires de liaison bioaffines est un porteur d'anticorps multifonctionnel pour renforcer une immunoprécipitation.

10. Procédé selon l'une quelconque des revendications précédentes, dans lequel la courbe de calibration est divisée en plus de deux régions partielles (A', B', C'), dans lequel la séquence d'entraînement est effectuée séparément, par groupe de deux régions partielles (A',B' ; A',C' ; B',C'), pour définir à chaque fois un algorithme de discrimination opérationnel et dans lequel, au cours de la séquence d'analyse, chacun des algorithmes de discrimination opérationnel est appliqué à chaque variation en fonction du temps S(t) mesurée selon l'étape c.a, pour associer les variables d'entrée X mesurées aux régions partielles A', B', C'.

Fig.1

Fig.2

Fig.3

EP 0 576 879 B1

# Fig.4